# EUROPEAN PATENT SPECIFICATION

(11) **EP 3 215 208 B1**
(45) Date of publication and mention of the grant of the patent: **09.08.2023**
(21) Application number: 15797517.8
(22) Date of filing: 03.11.2015
(51) Int. Cl.: A61M 25/00

(54) **MEDICAL DEVICE HAVING AN ATRAUMATIC DISTAL TIP**
MEDIZINISCHE VORRICHTUNG MIT ATRAUMATISCHER DISTALER SPITZE
DISPOSITIF MÉDICAL AYANT UN EMBOUT DISTAL ATRAUMATIQUE

(30) Priority: 07.11.2014 US 201462076914 P
(43) Date of publication of application: 13.09.2017
(73) Proprietor: Boston Scientific Scimed, Inc., Maple Grove, MN 55311 (US)
(72) Inventor: HAVERKOST, Patrick A., Corcoran, Minnesota 55374 (US); GROFF, JoeL N., Delano, Minnesota 55328 (US); WILLARD, Martin R., Burnsville, Minnesota 55337 (US); TASSONI, Anthony F., Jr., Ramsey, Minnesota 55303 (US)
(74) Representative: Vossius & Partner Patentanwälte Rechtsanwälte mbB
(86) International application number: PCT/US2015/058857
(87) International publication number: WO 2016/073498

(56) References cited:
- EP-A1- 2 436 417
- WO-A1-96/18431
- WO-A1-2010/123371
- US-A- 5 692 506
- US-A1- 2002 183 777
- US-A1- 2006 004 323
- US-A1- 2007 244 440

## Description

### TECHNICAL FIELD

The present disclosure pertains to medical devices, and methods for manufacturing medical devices. More particularly, the present disclosure pertains to elongated intracorporeal medical devices including a tubular body having an atraumatic, soft distal tip.

### BACKGROUND

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. US 5 692 506 A discloses a transnasal conduit for transesophageal echocardiography (TEE) probe insertion. A closed or closeable distal end facilitates insertion of the transnasal conduit into the patient during intubation. The open proximal end of the transnasal conduit may be bell shaped or flared to facilitate introduction of the probe. WO 96/18431 A1 discloses a catheter device having a catheter with a distal end and a proximal end, the distal end comprising one or more fins having an open position defining a large diameter and a closed position of small diameter. The catheter has a catheter body and a push tube sliding axially within the catheter body for manipulating the fins into the open and closed positions. WO 2010/123371 A1 discloses a guidewire system for insertion into a vascular system comprising a support catheter bounding a lumen for receiving at least a portion of the guidewire. The support catheter comprises a guidewire engagement structure for engaging the guidewire inside the lumen. US 2006/004323 A1 discloses a minimally invasive surgery system having a C-arm fluoroscope that is useable to visualize a first introducing device, a second introducing device and a working device. The introducing devices may be guide catheters or guide tubes and the working device may be a balloon catheter or other dilation catheter. US 2007/244440 A1 discloses a catheter including a wire guide lumen sized to receive a first wire guide of a first diameter and a tip lumen that extends in a distal direction from a first opening in communication with the wire guide lumen to a second opening. The first opening is sized to receive the first wire guide, and the second opening is sized to receive a second wire guide of a smaller diameter than the first wire guide. The catheter also includes one or more longitudinal expansion features capable of radially expanding the tip lumen to receive a wire guide of a diameter up to the first diameter through the second opening. US 2002/183777 A1 discloses a dilation catheter device comprising a shroud tube having a total preselected diameter effective for use in a preselected body lumen, the shroud tube having a strain relief tube extending therethrough which is adapted to reversibly form a flared, funnel-shape and a collapsed shape for contacting the surface of a dilation balloon. The configuration of the balloon can be altered by altering the position of contact of the distal end of the strain relief tube with the surface of the balloon. EP 2 436 417 A1 discloses an introducer sheath including a tubular body portion having a proximal region and a distal region and defining an internal lumen configured and dimensioned to slidably receive a catheter. A penetrating portion at a distal end of the tubular body has a first tapered configuration to enlarge an opening in a body tissue during distal advancement of the introducer sheath through the body tissue and a second expanded configuration to enable the passage of a distal end portion of a catheter through the penetrating portion. There is an ongoing need to provide alternative medical devices as well as alternative methods for manufacturing and using medical devices.

### SUMMARY

The present disclosure pertains to medical devices, and methods for manufacturing medical devices. More particularly, the present disclosure pertains to elongated intracorporeal medical devices including a tubular body incorporating other elements, and methods for manufacturing and using such devices.

The present invention relates to a delivery catheter for delivering a medical device to a target location within a patient's body defined by the features of claim 1. Variations and further developments are defined by the features of the dependent claims.

The delivery catheter includes an elongate main body and a distal tip extending distally of a portion of the main body. The distal tip is configured to transition from a first configuration in which an outer diameter of the distal tip is equal to or less than the outer diameter of the main body to a second configuration in which the outer diameter of the distal tip is greater than the outer diameter of the main body, wherein a wall thickness of the distal tip is greater than a wall thickness of the main body. The first configuration is a folded configuration in which the distal tip comprises two or more folds folded along a longitudinal axis of the main body.

Alternatively or additionally, a distal edge of the distal tip is rounded and is free of sharp edges.

Alternatively or additionally, the wall thickness of a region spaced proximally from a distal most end of the distal tip is greater than the wall thickness of the main body.

Alternatively or additionally, the wall thickness of a distal region of the distal tip is greater than the wall thickness of the main body.

Alternatively or additionally, the distal tip comprises up to eight folds spaced apart around an outer circumference of the distal tip.

Alternatively or additionally, the distal tip comprises three folds spaced apart from one another about an outer circumference of the distal tip.

Alternatively or additionally, in the second configuration the wall thickness of the distal tip substantially resists and/or prevents collapse of the distal tip during retrieval and/or repositioning of a medical device when the medical device contacts a distal most end of the main body.

Alternatively or additionally, in the first configuration the distal tip is configured to retain a medical device therein for delivery to the target location in the patient's body.

Alternatively or additionally, the distal tip comprises a polymer having a durometer of 30-40D.

The present invention also relates to a method of forming a soft, expandable distal tip at a distal end of a catheter body as defined by the features of independent claim 10. Variations and further developments are defined by the features of the dependent claims.

The method includes increasing a wall thickness in a selected region of a distal tip of the catheter body, the distal tip extending distally from a distal portion of the catheter body and comprising a soft, low durometer polymer, wherein the wall thickness in the selected region of the distal tip is greater than a wall thickness of the distal portion; and forming two or more folds in the distal tip, the two or more folds oriented along a longitudinal axis of the catheter body, wherein the distal tip is configured to transition from a folded configuration in which an outer diameter of the distal tip is equal to or less than an outer diameter of the catheter body to an unfolded configuration in which the outer diameter of the distal tip is greater than the outer diameter of the catheter body during delivery of a medical device.

Alternatively or additionally, forming the two or more folds comprises heating the distal tip to a temperature at or above a softening point of the polymer and cooling the distal portion including the distal tip of the elongate tubular body to a temperature below the softening point of the polymer.

Alternatively or additionally, the method further comprises increasing the wall thickness at a distal region of the distal tip and rounding a distal edge of the distal tip such that the distal edge is substantially free of sharp edges.

Alternatively or additionally, the method further comprises increasing a wall thickness in a region spaced proximally from a distal most end of the distal tip and rounding a distal edge such that the distal edge is substantially free of sharp edges.

Alternatively or additionally, the method further comprises loading a medical device into the distal tip.

Alternatively or additionally, in the first configuration the distal tip is configured to retain a leadless cardiac pacemaker therein for delivery to the target location in the patient's body.

In yet another example, a catheter includes a main body portion having an inner diameter and an outer diameter and a soft, distal tip extending distally from the main body portion. The distal tip comprising a rounded distal edge substantially free of sharp edges and wherein the distal tip is configured to transition from a folded configuration comprising two or more folds to an unfolded configuration, wherein in the unfolded configuration an outer diameter of the distal tip is greater than the outer diameter of the main body portion.

Alternatively or additionally, the distal tip has an outer diameter that is less than or equal to the outer diameter of the main body portion when in the folded configuration.

Alternatively or additionally, a wall thickness of a distal region of the distal tip is greater than a wall thickness of the main body portion.

Alternatively or additionally, the wall thickness of the distal tip is greatest in a distal region of the distal tip.

Alternatively or additionally the wall thickness of the distal tip is greatest in a region spaced proximally from a distal most end of the distal tip.

Alternatively or additionally, the wall thickness is such that in the unfolded configuration, the distal tip substantially resists collapse during retrieval and/or repositioning of a medical device when the medical device contacts a distal most edge of the main body portion.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description of various embodiments in connection with the accompanying drawings, in which:
Figure 1 is a schematic view of an exemplary catheter;
Figure 2 is a cross-sectional view of the catheter shown in Figure 1 taken through line 2e2;
Figures 3A and 3B are close-up, side schematic views of the distal portion of the catheter shown in Figure 1 including a distal tip;
Figures 4A and 4B are close-up, top-down schematic views of the distal portion of the catheter shown in Figure 1 including a distal tip;
Figures 5 and 6 are longitudinal, cross-sectional views of a distal portion of exemplary catheters including a distal tip;
Figures 7A-7D show a distal portion, including the distal tip, of a catheter during different stages of delivery of a medical device;
Figure 8 shows a distal tip of an exemplary catheter during retrieval of a medical device; and
Figure 9 is a flow chart of a method of manufacturing an exemplary catheter.

While the disclosure is amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the scope of the claims.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the disclosure.

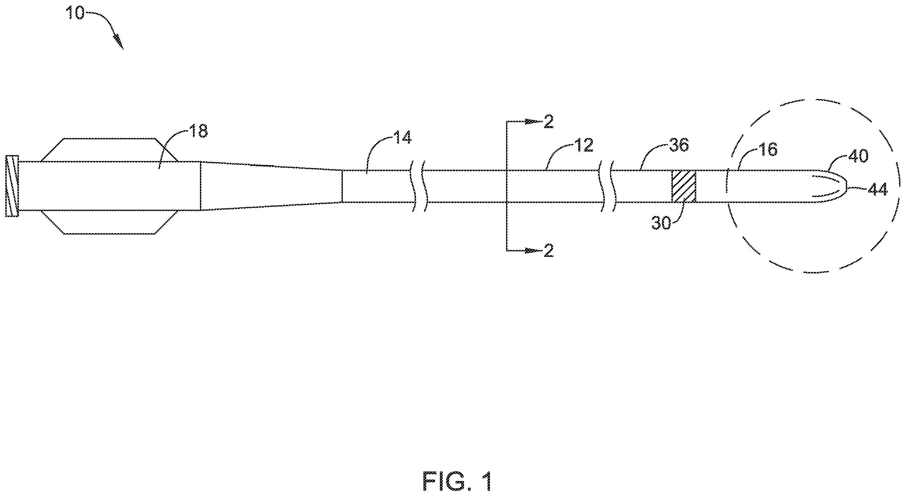

Figure 1 is a side schematic view of an exemplary catheter 10. Although catheter 10 is described as a delivery catheter, catheter 10 could be any other type of catheter including diagnostic or therapeutic catheters such as angioplasty balloon catheters, atherectomy catheters, stent delivery catheters, guide catheters, and the like, or any other suitable device. Furthermore, catheter 10 can generally include any device designed to pass through an opening or body lumen. For example, catheter 10 may include a sheath, endoscopic device, laparoscopic device, embolic protection device, guidewire and the like, or any other suitable device.

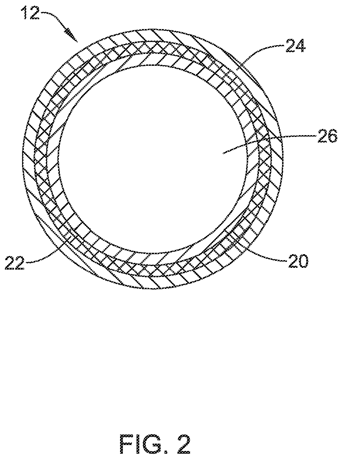

Catheter 10 includes an elongated catheter body 12 extending from a proximal end 14 to a distal end 16. Catheter 10 may include a lumen 26 (Figure 2) having an inner diameter ranging from 8 to about 15 French capable of passing a large payload therethrough. The lumen may extend from the proximal end 14 to the distal end 16. A hub 18 may be coupled to proximal end 14. In at least some embodiments, elongated catheter body 12 may include a plurality of layers. For example, Figure 2 illustrates that elongated catheter body 12 may include an inner liner or layer 20, a reinforcing layer 22, and an outer layer 24. Liner 20 may include lubricious material such as polytetrafluoroethylene (PTFE), etched PTFE, fluorinated ethylene propylene (FEP), or the like. Outer layer 24 may include one or more polymers such as polyether block amide, polyurethane, combinations or blends thereof, or the like. All of the layers 20, 22, 24 may extend along the full length of elongated catheter body 12. Alternatively, one or more of layers 20, 22, 24 may extend along only a portion of the length of elongated catheter body 12.

Reinforcing layer 22 may include a braid, coil, mesh, or other suitable reinforcement. In at least some embodiments, reinforcing layer 22 may include a polymeric braid. For example, reinforcing layer 22 may include an ultra-high molecular weight polyethylene braid. Other materials and/or reinforcements are contemplated including those disclosed herein. The presence of reinforcing layer 22 may provide elongated catheter body 12 with enhanced cut resistance, tear resistance, kink resistance, etc.

Alternatively and/or additionally, the elongated catheter body 12 may be manufactured such that it increases in flexibility along a length of the catheter body 12 from the proximal end 14 to the distal end 16. For example, in such an embodiment, a distal portion of the catheter body 12 may have a greater flexibility than a middle and/or proximal portion of the catheter body 12. Similarly, the middle portion may have less flexibility than the distal portion, but greater flexibility than the proximal portion. A variable flexibility profile may be achieved by manipulating the material properties and/or the mechanical/structural properties of the catheter body 12.

A number of different methods may be used to manufacture elongated catheter body 12. For example, liner 20 may be disposed on a mandrel. The mandrel may vary in size, depending on the intervention. For example, the mandrel may be a silver coated copper core or other suitable mandrel with an outer diameter in the range of about 0.01 to 0.05 inches (about 0.0254 to 0.127 centimeter), or about 0.02 to 0.04 inches (about 0.0508 to 0.1016 centimeter), or about 0.022 to 0.027 inches (about 0.05588 to 0.06858 centimeter) or so. In some embodiments, reinforcing layer 22 may be disposed along the outer surface of liner 20 and outer layer 24 may be disposed along the outer surface of reinforcing layer 22. In other embodiments, outer layer 24 may be disposed along the outer surface of liner 20 and reinforcing layer 22 may be disposed along the outer surface of outer layer 24. The process for disposing layers 20, 22, 24 onto the mandrel may include an extrusion process. When using an extrusion process, the medical device assembly may be subjected to extrusion temperatures in the range of about 100 to 200 °C, or about 120 to 190 °C, or about 140 to 170 °C. Under such conditions, reinforcing layer 22 may become embedded and/or at least partially embedded within outer layer 24. For example, at least a portion of outer layer 24 may be disposed radially outward of the outer surface of reinforcing layer 22. In some instances, reinforcing layer 22 may become disposed at or near the inner surface of outer layer 24 so that reinforcing layer 22 is essentially positioned between liner 20 and outer layer 24. In some of these and in other embodiments, portions of outer layer 24 may be interlocked with or otherwise disposed within the interstices of reinforcing layer 22. This may form or define a "composite layer" that includes both the material of reinforcing layer 22 and the material of outer layer 24. In at least some embodiments, the melting temperature of reinforcing layer 22 may be less than the melting temperature of liner 20, outer layer 24, or both. This is just one example.

Referring back to Figure 1, catheter 10 also includes a distal portion 36 including a soft distal tip 40. The distal tip 40 extends distally from the distal portion 36 of the catheter body 12 and may terminate at a distal most end 44 of the catheter 10. The distal tip 40 may be an atraumatic distal tip 40 that may not cause significant damage to a vessel wall, such as for example, the endothelium, during advancement of the catheter 10 to a desired location within a patient's body. In some cases, the catheter wall at the distal end 44 of the distal tip 40 may be rounded or smoothed such that the distal edge of the distal tip 40 is free of sharp edges to prevent the distal tip 40 from snagging or otherwise damaging tissue of the vessel wall through which the catheter 10 may be advanced. The distal tip 40 may encompass 50% or less of the total length of the catheter body 12. For example, the distal tip 40 may be 40%, 30%, 20%, 10%, 5%, 1%, etc. of the total length of the catheter body 12.

The distal portion 36 and/or the distal tip 40 may be formed from a soft polymeric material having a lower durometer and/or a greater flexibility than the remainder of the catheter body 12. The selected material from which the distal portion 36 and/or distal tip 40 is manufactured may be highly elastomeric and/or pliable. The hardness of plastics and/or elastomers is most commonly measured by the Shore t (Durometer) test or Rockwell hardness test. Both methods measure the resistance of plastics toward indentation and provide an empirical hardness value. Shore Hardness, using either the Shore A or Shore D scale (hence a low D or high A classification) is the preferred method for rubbers/elastomers and is also commonly used for 'softer' plastics such as polyolefins, fluoropolymers, and vinyls. The Shore A scale is used for 'softer' rubbers/elastomers while the Shore D scale is used for 'harder' ones. In addition, Shore hardness is often used as a proxy for flexibility for the specification of elastomers. In many cases, the polymer from which the distal tip may be fabricated includes a low D and/or a high A polymer. For example, the distal tip 40 may have a durometer hardness of 20-80D or 65-100A. In other examples, the distal tip 40 may have a durometer hardness of 30-40D. One exemplary polymer that may be used to fabricate the distal tip is a 3 5D Pebax. Other examples include high A polyurethanes or silicone rubber. These are just some examples.

Several different manufacturing techniques may be used to integrate the distal tip 40 into the catheter 10. In some cases, the distal tip 40 may fabricated separately and then bonded or fused to the catheter body 12. In other cases, the distal tip 40 may be co-extruded along with the outer layer 24 of the catheter body 12 using a bump extrusion process such that the higher durometer material used to form the outer layer 24 gradually transitions to the lower durometer distal tip 40 material from which the distal tip 40 may be formed. It will be generally understood that any reinforcing layer and/or liner 20 that may be present in the catheter body 12 such as, for example, reinforcing layer 22 and/or liner 20 may terminate prior to the distal tip 40.

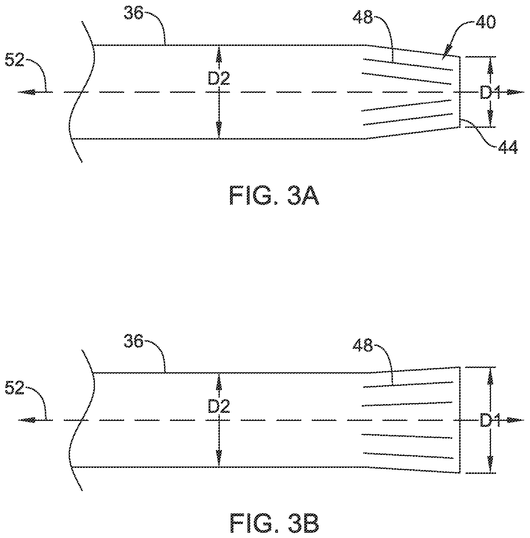

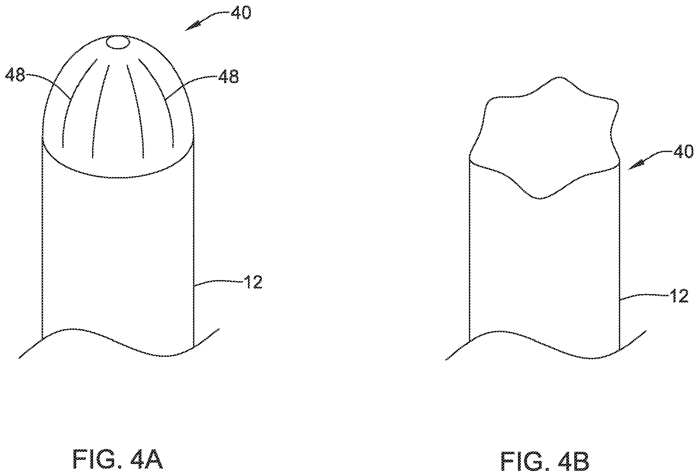

Additionally, the distal tip 40 is fabricated such that it is capable of transitioning from a first, folded configuration (shown in Figures 3A and 4A) to a second, unfolded configuration (shown in Figures 3B and 4B). In the first, folded configuration the distal tip 40 has an outer diameter Di that is substantially equal to or less than an outer diameter D₂ of the distal portion 36. Additionally, the distal tip 40 may be configured to retain a medical device therein for delivery to a target location within a patient's body in the first, folded configuration. The smaller outer diameter Di of the distal tip 40 may aid in retaining the medical device. Additionally, pleats or folds 48 in the distal tip 40 also aid in retaining the medical device. One such exemplary medical device is a leadless cardiac pacer (LCP). In the second, unfolded configuration (shown in Figures 3B and 4B), the distal tip has an outer diameter Di that is greater than an outer diameter D₂ of the distal portion 36. In some cases, in the second, unfolded configuration, the distal tip 40 may be flared, but this is not required.

The distal tip 40 is configured to transition from the first, folded configuration (Figures 3A and 4A) to the second, unfolded configuration (Figures 3B and 4B), e.g., upon delivery of a medical device or other payload (e.g. LCP) housed within the catheter 10 for delivery to a target location within the patient's body. To deliver the medical device to the target location, the catheter 10 may be either retracted in a proximal direction or the medical device may be pushed in a distal direction out of the distal end of the catheter 10 and hence, the distal tip 40, causing the distal tip 40 to transition from the folded configuration (Figures 3A and 4A) to the unfolded configuration (Figures 3B and 4B). The low durometer polymer from which the distal tip 40 may be fabricated may permit the distal tip 40 to easily transition from the unfolded configuration to the folded configuration. The distal tip 40 may be returned to the folded configuration from the unfolded configuration upon removal and/or withdrawal of the catheter 10 from the patient's body. In some cases, the catheter 10 may be retracted 10 into a guide sheath which may cause the distal tip 40 to return to the folded configuration from the unfolded configuration.

As shown in Figures 3A and 4A, in the folded configuration, the distal tip 40 includes two or more folds 48 oriented along a longitudinal axis 52 of the catheter body 12. The number of folds 48 may vary depending upon the overall size of the catheter 10 and its desired application. In some cases, the distal tip 40 may have as few as two folds 48 or as many as six, eight, ten or twelve. In one example, the distal tip 40 may have three folds 48. In some cases, the folds 48 may be spaced an equal distance from one another about an outer circumference of the distal tip 40. In other cases, the distance between the folds 48 may vary. Again, the spacing of the folds 48 from one another and the distance between each of the folds 48 may be altered based on the desired application and/or the type of medical device to be delivered.

The folds 48 can accommodate an increase in the amount of material used to form the distal tip 40 such that the distal tip 40 is capable of transitioning to an unfolded configuration having a greater outer diameter Di than the rest of the catheter body 12, while at the same time maintaining an outer diameter Di that is substantially equal to or less than an outer diameter D₂ of the rest of the catheter body when in the folded configuration. Additionally or alternatively, the folds 48 may accommodate an increased wall thickness in a selected region of the distal tip 40. The wall thickness in a selected region of the distal tip 40 may be greater than a wall thickness elsewhere in the distal tip 40 and/or the remainder of the catheter body 12. Because the distal tip 40 may be manufactured from a soft, low durometer polymer, the increase in wall thickness in a selected region of the tip 40 may help to prevent the distal tip 40 from collapsing or otherwise folding back in on itself when the distal tip 40 is in the unfolded, expanded configuration and is in contact with a medical device during repositioning and/or removal of the medical device from the patient's body. In addition, the distal tip 40 may be manufactured from a low durometer polymer to reduce and/or prevent trauma to the vessel wall and to facilitate transition of the distal tip from the folded, collapsed configuration to the unfolded, expanded configuration

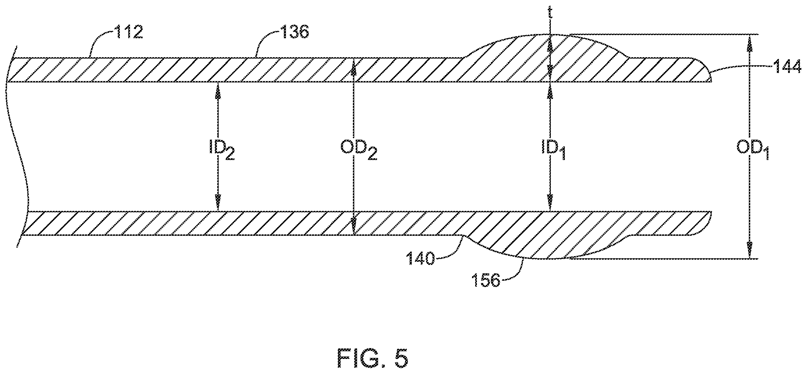

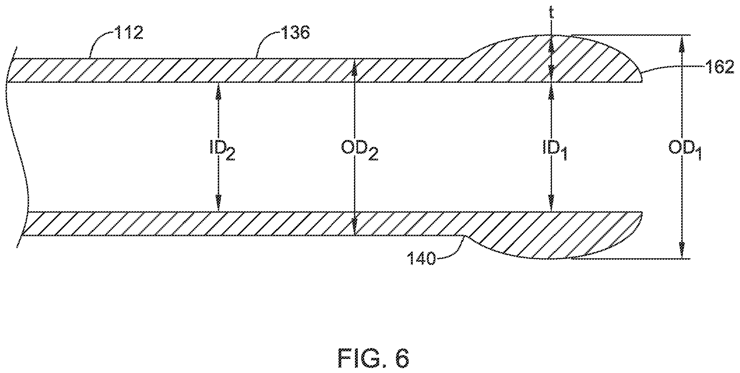

Figures 5 and 6 are longitudinal, cross-sectional views of a distal portion 136 of a catheter body 112 and distal tip 140 having an increased wall thickness t in selected regions 156, 162 of the tip 140. For convenience and ease of understanding, the folds are not shown. However, it will be generally understood that the increased wall thickness in a selected region of the distal tip 140 may be in addition to any folds or folds formed therein, and that the increased wall thickness may be accommodated by the folds formed in the distal tip.

Figure 5 shows an increased wall thickness t in a region 156 spaced proximally from the distal most end 144 of the distal tip 140. For example, the region 156 may be spaced 1 mm to 50 mm from the distal most end 144. In other examples, the region 156 may be spaced 1 mm to 20 mm, 1 mm to 10 mm, or 1 mm to 5 mm from the distal most end 144. Figure 6 shows an increased wall thickness t in a distal region 162 of the tip 140. Additionally or alternatively, the wall thickness t may be increased at a distal edge of the tip 140. In some examples, the wall thickness t may be increased in a selected region 156 or 162 such that the outer diameter ODi of the selected region 156 or 162 is greater than an outer diameter OD₂ of the remaining portion of the distal tip 140 and/or the distal portion 136 of the catheter body 112 from which the distal tip 140 extends. However, despite the increase in outer diameter at the selected region 156 or 162 of the distal tip 140, in some examples, the inner diameter ID₁ of the distal tip 140 may be substantially equal to the inner diameter ID₂ of the distal portion 136 and the catheter body 112, but this is not required in all embodiments. Maintaining a constant inner diameter between a main portion of the catheter body 112, including the distal portion 136, and the distal tip 140 may facilitate accommodation of a variety of medical devices to be passed through the catheter body 112 and into the distal tip 140 for delivery and deployment at a target location within the patient's body.

As previously discussed herein, the increased wall thickness in the selected region 156 or 162 of the distal portion 136 may be accommodated by any folds 48 (shown in Figures 3A-4B) formed in the tip 140. The folds permit the tip 140 having an increased wall thickness in a selected region to be collapsed into a folded configuration such that the outer diameter of the distal tip 140, in the folded configuration is substantially equal to or less than the outer diameter of the catheter body 112. The tip's ability to be placed into a folded configuration having an outer diameter that is substantially equal to or less than the outer diameter of the catheter body 112 may permit a somewhat larger tip 40, 140 to be passed and/or retracted through an introducer sheath in use during a procedure.

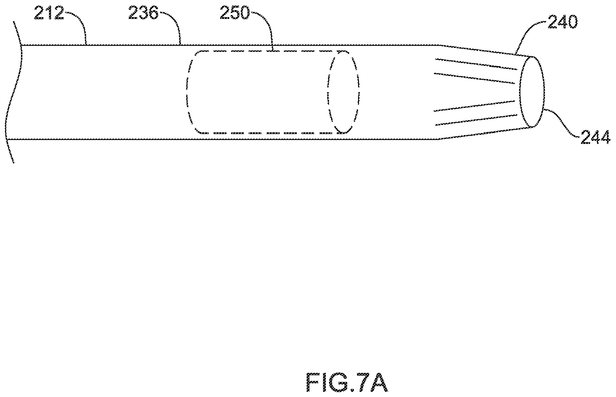

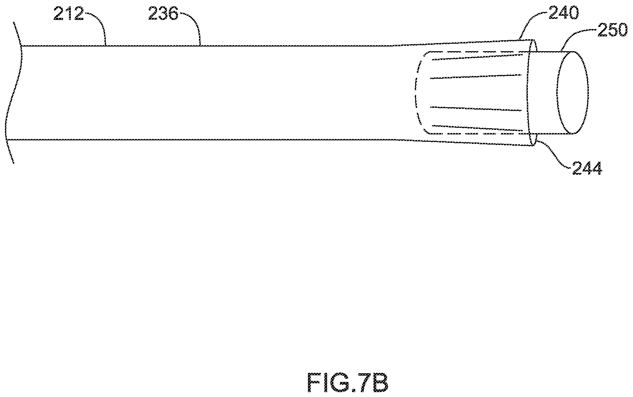

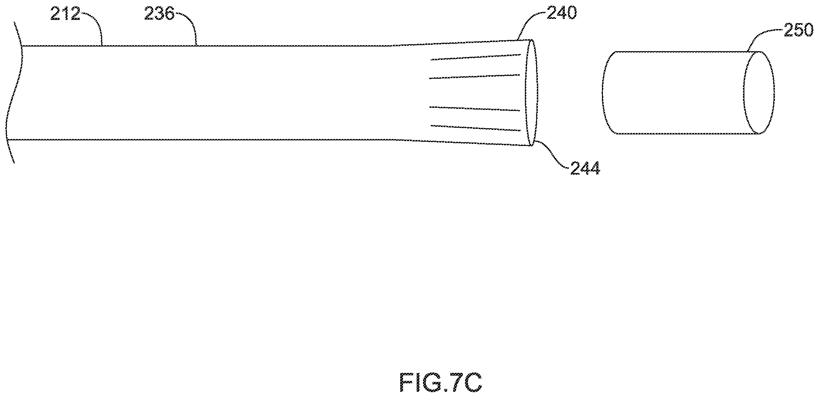

Figures 7A-7D show a distal portion 236 and the distal tip 240 of a catheter body 212 during different stages of delivery of an exemplary medical device 250 such as, for example, a leadless cardiac pacer (LCP). As shown in Figure 7A, the medical device 250 may be contained within the distal portion 236 of the catheter body 212 and/or at least partially within the distal tip 240 during delivery of the medical device 250 to a target location of a patient's body. In some cases, the medical device 250 may be back-loaded into the distal portion 236 and/or distal tip 240 of the catheter body 212. Figure 7B shows the distal portion 236 and distal tip 240 during partial delivery of the medical device 250. The medical device 250 may be delivered to a target site within a patient's body by retraction of the catheter in a proximal direction (i.e. towards the clinician performing the procedure) or by being pushed in a distal direction out of a distal most end 244 of the catheter body 212. As the medical device 250 passes through the open distal most end 244 of the catheter body, it causes the distal tip 240 to transition from the folded configuration shown in Figure 7A to the unfolded configuration shown in Figure 7C. Figure 7C shows the distal portion 236 and distal tip 240 after delivery of the medical device 250 to the target location within the patient's body. As shown in Figure 7C, the distal tip 240 may remain in the unfolded configuration after delivery of the medical device 250.

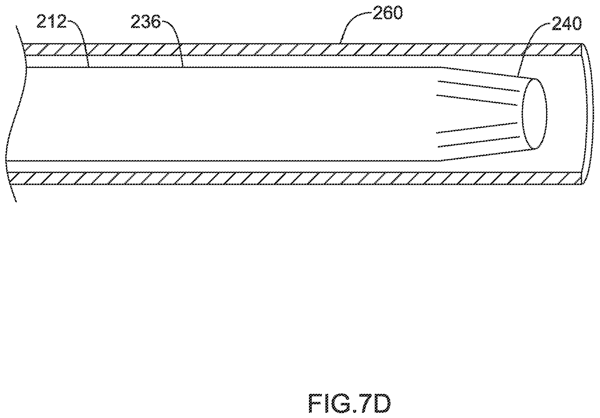

Distal tip 240 may be returned to the folded configuration using an outer sheath 260, as shown in Figure 7D. In some cases, an outer sheath 260 may either be advanced in a distal direction over the catheter including the distal portion 236 and distal tip 240, causing the distal tip 240 to at least partially return to the folded configuration. In other cases, the catheter may be withdrawn in a proximal direction into the outer sheath 260, causing the distal tip 240 to at least partially return to the folded configuration. The catheter may then be removed from the patient's body.

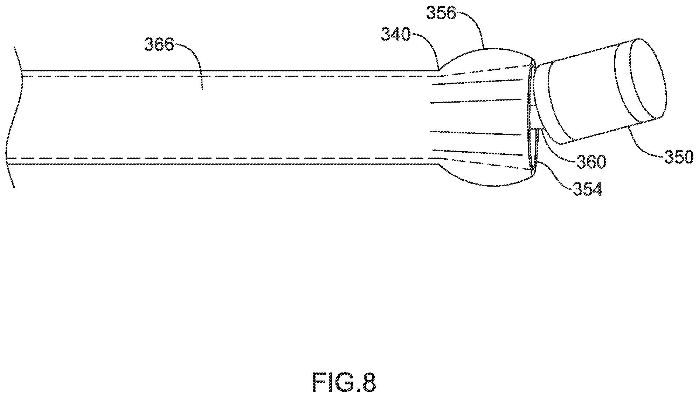

Figure 8 is a schematic view of a distal tip 340 of an exemplary catheter, as described herein, during retrieval of an exemplary medical device 350. The medical device 350 may be retrieved such that it may be repositioned or extracted from the patient's body. The increased wall thickness in region 356 and/or increased outer diameter of the distal tip 340, as described herein, may resist, reduce and/or prevent the distal tip 340 from collapsing or folding back in on itself when an edge 354 of the distal tip 340 contacts the medical device 350 and more particularly, when the edge 354 of the distal tip 340 contacts the medical device 350 at an angle, such as may occur during retrieval and/or repositioning of the medical device 350. In addition, because the medical device 350 may be tethered or a secondary tool 360 may be used to couple to the medical device 350, reduction and/or prevention of collapse of the distal tip 340 may facilitate alignment of the medical device 350 with the catheter lumen 366 such that the medical device 350 can be withdrawn into the catheter lumen 366 to facilitate repositioning or removal of the device 350.

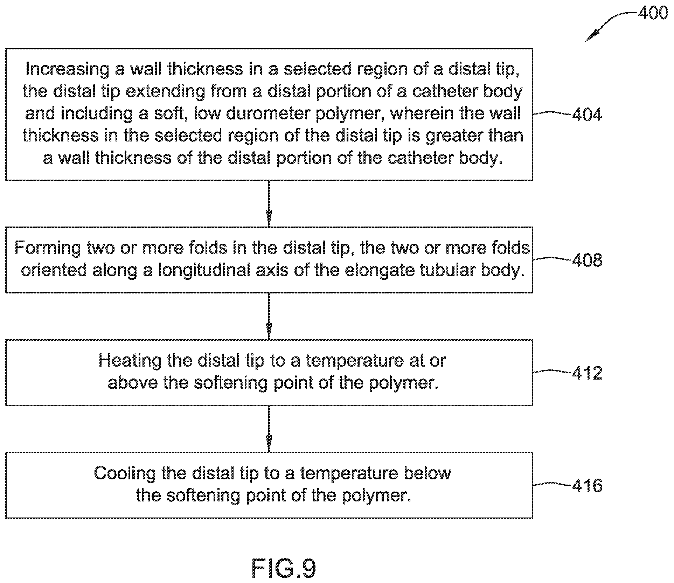

Figure 9 is a flow chart of a method 400 that may be used to fabricate a catheter including a distal tip having two or more folds as described herein. As described herein, a distal tip may be formed from a polymeric material having a lower durometer and/or a greater flexibility than the remainder of the catheter body. The distal tip may be formed using an extrusion process. The distal tip may be fabricated separately from the rest of the catheter body and then fused or bonded to a distal end of the catheter body or it may be co-extruded with at least an outer layer of the catheter body as discussed previously herein. Regardless of the manufacturing method used to create the tip, a wall thickness in a selected region of the distal tip is increased (Block 404). This may be accomplished during an extrusion process used to form the distal tip. The wall thickness may be increased in a region spaced proximally from a distal most end or a distal region of the distal tip. In some cases, the outer diameter of the distal tip may be increased while the inner diameter is maintained such that it is substantially equal to an inner diameter of lumen extending within the catheter body. Next, two or more folds are formed in the distal tip (Block 408). The two or more folds may be formed in the distal tip by applying a crimping tool over the distal tip and heating the distal tip to a temperature at or above a softening point of the polymer used to form the distal tip (Block 412). The distal tip assembly including the crimping tool is then cooled to set the two or more folds (Block 416). The distal tip assembly may be cooled to a temperature below the softening point of the polymer used to form the distal tip. In some case, cooling may be accomplished by cooling the assembly at room temperature or by plunging the assembly in a cool water bath or an ice bath. Once the distal tip has been cooled for a sufficient amount of time, the crimping tool may be removed.

Those skilled in the art will recognize that the present disclosure may be manifested in a variety of forms other than the specific embodiments described and contemplated herein. Accordingly, departure in form and detail may be made without departing from the scope of the present disclosure as described in the appended claims.

## Claims

1. A delivery catheter (10) for delivering a medical device (250; 350) to a target location within a patient's body, the catheter comprising:
an elongate main body (12; 112; 212); and
a distal tip (40; 140; 240; 340) extending distally of a portion of the main body (12; 112; 212), wherein the distal tip (40; 140; 240; 340) is configured to transition from a first configuration in which an outer diameter (D₁) of the distal tip (40; 140; 240; 340) is equal to or less than the outer diameter (D₂) of the main body (12; 112; 212) to a second configuration in which the outer diameter (D₁) of the distal tip (40; 140; 240; 340) is greater than the outer diameter (D₂) of the main body (12; 112; 212) and wherein a wall thickness of the distal tip (40; 140; 240; 340) is greater than a wall thickness of the main body (12; 112; 212),
wherein the first configuration is a folded configuration in which the distal tip (40; 140; 240; 340) comprises two or more folds (48) folded along a longitudinal axis (52) of the main body (12; 112; 212).

2. The delivery catheter of claim 1, wherein a distal edge of the distal tip (40; 140; 240; 340) is rounded and is free of sharp edges.

3. The delivery catheter of any one of claims 1 or 2, wherein the wall thickness of a region (156) spaced proximally from a distal most end (144) of the distal tip (140) is greater than the wall thickness of the main body (112).

4. The delivery catheter of any one of claims 1 or 2, wherein the wall thickness of a distal region (162) of the distal tip (140) is greater than the wall thickness of the main body (112).

5. The delivery catheter of claim 1, wherein the distal tip (40; 140; 240; 340) comprises up to eight folds (48) spaced apart around an outer circumference of the distal tip (40; 140; 240; 340).

6. The delivery catheter of claim 1, wherein the distal tip (40; 140; 240; 340) comprises three folds (48) spaced apart from one another about an outer circumference of the distal tip (40; 140; 240; 340).

7. The delivery catheter of any one of claims 1-6, wherein in the second configuration the wall thickness of the distal tip (240; 340) substantially resists and/or prevents collapse of the distal tip (240; 340) during retrieval and/or repositioning of a medical device (250; 350) when the medical device (250; 350) contacts a distal most end (244) of the main body (212).

8. The delivery catheter of any one of claims 1-7, wherein in the first configuration the distal tip (40; 140; 240; 340) is configured to retain a medical device (250; 350) therein for delivery to the target location in the patient's body.

9. The delivery catheter of any one of claims 1-8, wherein the distal tip (40; 140; 240; 340) comprises a polymer having a durometer of 30-40D.

10. A method of forming a soft, expandable distal tip (40; 140; 240; 340) at a distal end of a catheter body (12; 112; 212) comprising:
increasing a wall thickness in a selected region of a distal tip (40; 140; 240; 340) of the catheter body (12; 112; 212), the distal tip (40; 140; 240; 340) extending distally from a distal portion of the catheter body (12; 112; 212) and comprising a soft, low durometer polymer, wherein the wall thickness in the selected region of the distal tip (40; 140; 240; 340) is greater than a wall thickness of the distal portion; and
forming two or more folds (48) in the distal tip (40; 140; 240; 340), the two or more folds (48) oriented along a longitudinal axis (52) of the catheter body (12; 112; 212), wherein the distal tip (40; 140; 240; 340) is configured to transition from a folded configuration in which an outer diameter (D₁) of the distal tip (40; 140; 240; 340) is equal to or less than an outer diameter (D₂) of the catheter body (12; 112; 212) to an unfolded configuration in which the outer diameter (D₁) of the distal tip (40; 140; 240; 340) is greater than the outer diameter (D₂) of the catheter body (12; 112; 212) during delivery of a medical device (250; 350).

11. The method of claim 10, wherein forming the two or more folds (48) comprises heating the distal tip (40; 140; 240; 340) to a temperature at or above a softening point of the polymer and cooling the distal portion including the distal tip (40; 140; 240; 340) of the catheter body (12; 112; 212) to a temperature below the softening point of the polymer.

12. The method of any one of claims 10 or 11, further comprising increasing the wall thickness at a distal region (162) of the distal tip (140) and rounding a distal edge of the distal tip (140) such that the distal edge is substantially free of sharp edges.

13. The method of any one of claims 10 or 11, further comprising increasing a wall thickness in a region (156) spaced proximally from a distal most end (144) of the distal tip (140) and rounding a distal edge such that the distal edge is substantially free of sharp edges.

14. The method of any one of claims 10-13, further comprising loading a medical device (250; 350) into the distal tip (240; 340).

## Patentansprüche

1. Zuführungskatheter (10) zum Zuführen einer medizinischen Vorrichtung (250; 350) zu einem Zielort in einem Körper eines Patienten, wobei der Katheter aufweist:
einen langgestreckten Hauptkörper (12; 112; 212); und
eine distale Spitze (40; 140; 240; 340), die sich distal von einem Abschnitt des Hauptkörpers (12; 112; 212) erstreckt, wobei die distale Spitze (40; 140; 240; 340) dazu eingerichtet ist, von einer ersten Konfiguration, in der ein Außendurchmesser (D₁) der distalen Spitze (40; 140; 240; 340) gleich oder kleiner als der Außendurchmesser (D₂) des Hauptkörpers (12; 112; 212) ist, in eine zweite Konfiguration, in der der Außendurchmesser (D₁) der distalen Spitze (40; 140; 240; 340) größer als der Außendurchmesser (D₂) des Hauptkörpers (12; 112; 212) ist, überzugehen und wobei eine Wanddicke der distalen Spitze (40; 140; 240; 340) größer als eine Wanddicke des Hauptkörpers (12; 112; 212) ist,
wobei die erste Konfiguration eine gefaltete Konfiguration ist, in der die distale Spitze (40; 140; 240; 340) zwei oder mehr Falten (48) aufweist, die entlang einer Längsachse (52) des Hauptkörpers (12; 112; 212) gefaltet sind.

2. Zuführungskatheter nach Anspruch 1, wobei eine distale Kante der distalen Spitze (40; 140; 240; 340) abgerundet und frei von scharfen Kanten ist.

3. Zuführungskatheter nach einem der Ansprüche 1 oder 2, wobei die Wanddicke eines Bereichs (156), der proximal von einem distalsten Ende (144) der distalen Spitze (140) beabstandet ist, größer ist als die Wanddicke des Hauptkörpers (112).

4. Zuführungskatheter nach einem der Ansprüche 1 oder 2, wobei die Wanddicke eines distalen Bereichs (162) der distalen Spitze (140) größer ist als die Wanddicke des Hauptkörpers (112).

5. Zuführungskatheter nach Anspruch 1, wobei die distale Spitze (40; 140; 240; 340) bis zu acht Falten (48) aufweist, die um einen Außenumfang der distalen Spitze (40; 140; 240; 340) voneinander beabstandet sind.

6. Zuführungskatheter nach Anspruch 1, wobei die distale Spitze (40; 140; 240; 340) drei Falten (48) aufweist, die um einen Außenumfang der distalen Spitze (40; 140; 240; 340) voneinander beabstandet sind.

7. Zuführungskatheter nach einem der Ansprüche 1 bis 6, wobei in der zweiten Konfiguration die Wanddicke der distalen Spitze (240; 340) einem Kollabieren der distalen Spitze (240; 340) während der Entnahme und/oder Neupositionierung einer medizinischen Vorrichtung (250; 350) im Wesentlichen widersteht und/oder dieses verhindert, wenn die medizinische Vorrichtung (250; 350) ein distalstes Ende (244) des Hauptkörpers (212) berührt.

8. Zuführungskatheter nach einem der Ansprüche 1 bis 7, wobei in der ersten Konfiguration die distale Spitze (40; 140; 240; 340) dazu eingerichtet ist, darin eine medizinische Vorrichtung (250; 350) zur Zuführung zum Zielort im Körper des Patienten zu halten.

9. Zuführungskatheter nach einem der Ansprüche 1 bis 8, wobei die distale Spitze (40; 140; 240; 340) ein Polymer mit einer Härte von 30-40D aufweist.

10. Verfahren zum Bilden einer weichen, dehnbaren distalen Spitze (40; 140; 240; 340) an einem distalen Ende eines Katheterkörpers (12; 112; 212), das aufweist:
Erhöhen einer Wanddicke in einem ausgewählten Bereich einer distalen Spitze (40; 140; 240; 340) des Katheterkörpers (12; 112; 212), wobei sich die distale Spitze (40; 140; 240; 340) distal von einem distalen Abschnitt des Katheterkörpers (12; 112; 212) erstreckt und ein weiches Polymer mit niedriger Härte aufweist, wobei die Wanddicke in dem ausgewählten Bereich der distalen Spitze (40; 140; 240; 340) größer ist als eine Wanddicke des distalen Abschnitts; und
Bilden von zwei oder mehr Falten (48) in der distalen Spitze (40; 140; 240; 340), wobei die zwei oder mehr Falten (48) entlang einer Längsachse (52) des Katheterkörpers (12; 112; 212) ausgerichtet sind, wobei die distale Spitze (40; 140; 240; 340) dazu eingerichtet ist, während der Zuführung einer medizinischen Vorrichtung (250; 350) von einer gefalteten Konfiguration, in der ein Außendurchmesser (D₁) der distalen Spitze (40; 140; 240; 340) gleich oder kleiner als ein Außendurchmesser (D₂) des Katheterkörpers (12; 112; 212) ist, in eine entfaltete Konfiguration, in der der Außendurchmesser (D₁) der distalen Spitze (40; 140; 240; 340) größer als der Außendurchmesser (D₂) des Katheterkörpers (12; 112; 212) ist, überzugehen.

11. Verfahren nach Anspruch 10, wobei das Bilden der zwei oder mehr Falten (48) ein Erwärmen der distalen Spitze (40; 140; 240; 340) auf eine Temperatur bei oder über einem Erweichungspunkt des Polymers und ein Abkühlen des distalen Abschnitts einschließlich der distalen Spitze (40; 140; 240; 340) des Katheterkörpers (12; 112; 212) auf eine Temperatur unter dem Erweichungspunkt des Polymers aufweist.

12. Verfahren nach einem der Ansprüche 10 oder 11, das ferner ein Erhöhen der Wanddicke in einem distalen Bereich (162) der distalen Spitze (140) und ein Abrunden einer distalen Kante der distalen Spitze (140), so dass die distale Kante im Wesentlichen frei von scharfen Kanten ist, aufweist.

13. Verfahren nach einem der Ansprüche 10 oder 11, das ferner ein Erhöhen der Wanddicke in einem Bereich (156), der proximal von einem distalsten Ende (144) der distalen Spitze (140) beabstandet ist, und ein Abrunden einer distalen Kante, so dass die distale Kante im Wesentlichen frei von scharfen Kanten ist, aufweist.

14. Verfahren nach einem der Ansprüche 10 bis 13, das ferner ein Laden einer medizinischen Vorrichtung (250; 350) in die distale Spitze (240; 340) aufweist.

## Revendications

1. Cathéter de mise en place (10) pour mettre en place un dispositif médical (250; 350) à un emplacement cible dans le corps d'un patient, le cathéter comprenant:
un corps principal allongé (12; 112; 212); et
une pointe distale (40; 140; 240; 340) s'étendant de manière distale d'une partie du corps principal (12; 112; 212), dans lequel la pointe distale (40; 140; 240; 340) est configurée pour passer d'une première configuration dans laquelle un diamètre externe (D₁) de la pointe distale (40; 140; 240; 340) est égal ou inférieur au diamètre externe (D₂) du corps principal (12; 112; 212) à une seconde configuration dans laquelle le diamètre externe (D₁) de la pointe distale (40; 140; 240; 340) est supérieur au diamètre externe (D₂) du corps principal (12; 112; 212) et dans lequel une épaisseur de paroi de la pointe distale (40; 140; 240; 340) est supérieure à une épaisseur de paroi du corps principal (12; 112; 212),
dans lequel la première configuration est une configuration repliée dans laquelle la pointe distale (40; 140; 240; 340) comprend deux ou plusieurs plis (48) repliés le long d'un axe longitudinal (52) du corps principal (12; 112; 212).

2. Cathéter de mise en place selon la revendication 1, dans lequel un bord distal de la pointe distale (40; 140; 240; 340) est arrondi et est dépourvu d'arêtes vives.

3. Cathéter de mise en place selon l'une quelconque des revendications 1 ou 2, dans lequel l'épaisseur de paroi d'une région (156) espacée de manière proximale d'une extrémité la plus distale (144) de la pointe distale (140) est supérieure à l'épaisseur de paroi du corps principal (112).

4. Cathéter de mise en place selon l'une quelconque des revendications 1 ou 2, dans lequel l'épaisseur de paroi d'une région distale (162) de la pointe distale (140) est supérieure à l'épaisseur de paroi du corps principal (112).

5. Cathéter de mise en place selon la revendication 1, dans lequel la pointe distale (40; 140; 240; 340) comprend jusqu'à huit plis (48) les uns des autres espacés autour d'une circonférence externe de la pointe distale (40; 140; 240; 340).

6. Cathéter de mise en place selon la revendication 1, dans lequel la pointe distale (40; 140; 240; 340) comprend trois plis (48) espacés les uns des autres autour d'une circonférence externe de la pointe distale (40; 140; 240; 340).

7. Cathéter de mise en place selon l'une quelconque des revendications 1 à 6, dans lequel, dans la seconde configuration, l'épaisseur de paroi de la pointe distale (240; 340) résiste sensiblement à et/ou empêche l'affaissement de la pointe distale (240; 340) pendant la récupération et /ou le repositionnement d'un dispositif médical (250; 350) lorsque le dispositif médical (250; 350) entre en contact avec une extrémité la plus distale (244) du corps principal (212).

8. Cathéter de mise en place selon l'une quelconque des revendications 1 à 7, dans lequel, dans la première configuration, la pointe distale (40; 140; 240; 340) est configurée pour retenir un dispositif médical (250; 350) à l'intérieur pour la mise en place à l'emplacement cible dans le corps du patient.

9. Cathéter de mise en place selon l'une quelconque des revendications 1 à 8, dans lequel la pointe distale (40; 140; 240; 340) comprend un polymère ayant une dureté de 30 à 40D.

10. Procédé de formation d'une pointe distale expansible souple (40; 140; 240; 340) à une extrémité distale d'un corps de cathéter (12; 112; 212) comprenant:
l'augmentation d'une épaisseur de paroi dans une région choisie d'une pointe distale (40; 140; 240; 340) du corps de cathéter (12; 112; 212), la pointe distale (40; 140; 240; 340) s'étendant de manière distale d'une partie distale du corps de cathéter (12; 112; 212) et comprenant un polymère souple à faible dureté, dans lequel l'épaisseur de paroi dans la région choisie de la pointe distale (40; 140; 240; 340) est supérieure à une épaisseur de paroi de la partie distale; et
la formation de deux ou plusieurs plis (48) dans la pointe distale (40; 140; 240; 340), les deux ou plusieurs plis (48) orientés le long d'un axe longitudinal (52) du corps de cathéter (12; 112; 212), dans lequel la pointe distale (40; 140; 240; 340) est configurée pour passer d'une configuration repliée dans laquelle un diamètre externe (D₁) de la pointe distale (40; 140; 240; 340) est égal ou inférieur à un diamètre externe (D₂) du corps de cathéter (12; 112; 212) à une configuration dépliée dans laquelle le diamètre externe (D₁) de la pointe distale (40; 140; 240; 340) est supérieur au diamètre externe (D₂) du corps de cathéter (12; 112; 212) pendant la mise en place d'un dispositif médical (250; 350).

11. Procédé selon la revendication 10, dans lequel la formation des deux ou plusieurs plis (48) comprend le chauffage de la pointe distale (40; 140; 240; 340) à une température égale ou supérieure à un point de ramollissement du polymère et le refroidissement de la partie distale incluant la pointe distale (40; 140; 240; 340) du corps de cathéter (12; 112; 212) à une température inférieure au point de ramollissement du polymère.

12. Procédé selon l'une quelconque des revendications 10 ou 11, comprenant en outre l'augmentation de l'épaisseur de paroi au niveau d'une région distale (162) de la pointe distale (140) et l'arrondissement d'un bord distal de la pointe distale (140) de sorte que le bord distal est sensiblement dépourvu d'arêtes vives.

13. Procédé selon l'une quelconque des revendications 10 ou 11, comprenant en outre l'augmentation d'une épaisseur de paroi dans une région (156) espacée de manière proximale d'une extrémité la plus distale (144) de la pointe distale (140) et l'arrondissement d'un bord distal de sorte que le bord distal est sensiblement dépourvu d'arêtes vives.

14. Procédé selon l'une quelconque des revendications 10 à 13, comprenant en outre le chargement d'un dispositif médical (250; 350) dans la pointe distale (240; 340).
